# EUROPEAN PATENT SPECIFICATION

(11) **EP 4 181 818 B1**
(45) Date of publication and mention of the grant of the patent: **02.04.2025**
(21) Application number: 21763262.9
(22) Date of filing: 06.08.2021
(51) Int. Cl.: A61C 7/08, A61F 5/56, A63B 71/08, A61K 9/00, A61Q 11/00, B29C 48/00

(54) **DENTAL APPLIANCE AND PROCESS FOR MAKING A THERMOPLASTIC FOIL**
ZAHNÄRZTLICHE VORRICHTUNG UND VERFAHREN ZUR HERSTELLUNG EINER THERMOPLASTISCHEN FOLIE
APPAREIL DENTAIRE ET PROCÉDÉ DE FABRICATION PROCÉDÉ DE FABRICATION D'UN FILM THERMOPLASTIQUE

(30) Priority: 14.08.2020 EP 20191189
(43) Date of publication of application: 24.05.2023
(60) Divisional application: 24215594.3
(73) Proprietor: Bussmedical AG, 6004 Luzern (CH)
(72) Inventor: TÖPPER, Tino, 79115 Freiburg (DE); OSMANI, Bekim, 4056 Basel (CH); DARD, Elise, 4055 Basel (CH)
(74) Representative: Mertzlufft-Paufler, Cornelius
(86) International application number: PCT/EP2021/072046
(87) International publication number: WO 2022/033985

(56) References cited:
- WO-A1-2021/102260
- US-A1- 2002 083 955
- US-A1- 2005 100 853
- US-A1- 2008 211 123
- US-A1- 2008 293 007
- US-A1- 2013 259 920
- US-A1- 2014 298 886
- US-A1- 2018 344 579

## Description

The present disclosure relates to a process for making a thermoplastic functional foil comprising a core of thermoplastic material, in particular a core made from a thermoplastic polymer, and a thermoplastic coating at least partially covering the core.

This foil is particularly suited for intra-oral use as a dental appliance to be worn inside the oral cavity on the teeth, since such an appliance may be easily formed from such a foil. Hence the disclosure also concerns a dental appliance fabricated, in particular formed, most preferably thermoformed, from such a functional foil. The dental appliance may be used as an orthodontic appliance or a protective appliance for protection of teeth and/or gingiva of the user of the appliance.

In the past, intraoral orthodontic devices have been used in orthodontic therapy for realigning teeth. In this specific application case, the devices are typically designed as a dental splint, which is applied to teeth over several hours. The dental splint exerts forces on the individual teeth of the patient, which results in a realignment of the teeth in directions of desired teeth positions. For this purpose, typically a number of dental splints, which vary slightly from each other in shape, are worn by the patient consecutively to realign the teeth step-wise.

Further known applications of such intraoral orthodontic devices, in particular dental splints, are bleaching of teeth using bleaching pastes which are applied to the teeth by the device, or use of such devices for treating snoring overnight. Intraoral appliances such as orthodontic devices are also used for avoiding clenching and grinding. Chronic teeth clenching and teeth grinding can cause overuse of the muscles controlling the lower jaw, leading to pain from those muscles. The load on the joint itself can also cause changes inside the joint, leading to pain and limited opening of the mouth.

In all of these applications, providing a high wearing comfort of the device is of central importance for a high acceptance of the treatment by the patient and hence overall success of the therapy. But also when a dental appliance is worn purely for protection of teeth, a high wearing comfort is an important quality.

US 2005/10853 A1 discloses an appliance for use in an oral cavity comprising a polymeric shell. The appliance is fabricated by thermoforming a sheet which is shaped from a mixture of thermoplastic polymers.

US 2013/259920 A1 describes a process for fabrication of a bilayered laminate from two thermoplastic hot-melts by coextrusion with the aim of forming a uniform transverse cross-section of the laminate that can be used as a buccal dosage form. To this purpose, both thermoplastic components are heated to a molten state and forced through an orifice where the extruded laminate is formed into its final shape and which solidifies upon cooling. No lamination step is thus required to form the two-layer laminate.

US 2008/293007 A1 also discusses methods for intra-oral drug delivery and proposes to attach bio-active substances to an oral appliance. In detail, it is suggested to adhere a layer with embedded agent to a portion of a surface of the oral appliance. The layer can thus later release the agent into the mouth of the patient, when the patient is wearing the appliance in his mouth.

The invention is as defined in the independent claims.

In this context, the invention aims at further enhancing the functionality and biocompatibility of such dental appliances and - in relation to this fundamental aim - at providing an efficient method for fabricating such devices.

In accordance with the present invention, a process is provided according to claim 1, which solves the afore-mentioned problem. In particular, the invention proposes a process for making a thermoplastic functional foil as described at the beginning featuring a thermoplastic core and a thermoplastic coating at least partially, but preferably fully, covering the core. This process comprises the following steps: providing a carrier liquid comprising an organic polymer, which may preferably be a thermoplastic polymer; adding an agent, releasable from the coating in aqueous environment, to the carrier liquid prior to forming the coating; applying the carrier liquid in the form of a liquid coating onto the solid core, which has the form of a core foil; and solidifying the liquid coating afterwards on the core foil to form a solid thermoplastic coating covering the core.

Particularly suitable materials for the core are polyethylene terephthalate (PET), in particular PETG, polycarbonate (PC), and polyurethane (PU). These materials may also be used in recycled form (rPET, rPU, rPET, rPETG).

In other words, the invention suggests to apply the coating in the form of liquid coating onto a solid core, which has the form of a core foil, in particular onto both sides of such a core foil, and to solidify the liquid coating afterwards on the core to form a solid thermoplastic coating covering the core.

Through this process, a highly uniform and homogenous coating of the core can be achieved, and both the core and the coating can be thermoformed together in a later process step, as will be explained in greater detail below. Another great advantage of this liquid coating approach is that the coating can provide new functionalities to the foil and dental appliances formed from the foil. In particular, as will be explained in greater detail below, an agent may be mixed into the carrier liquid forming the coating prior to solidifying the coating on the core.

A carrier melt may also be applied onto a solid core to form a functional coating.

As will become clear below, the coating may form a thermoplastic cap layer of a dental appliance fabricated from the foil. For particular applications, for example when antimicrobial protection is to be provided, it can be beneficial if the cap layer fully covers the core of the device, and hence the thermoplastic coating may fully cover the core of the functional foil.

According to the invention, there exist further advantageous examples solving the aforementioned problems, which are described in the following:
For example, the agent may be added to the carrier liquid in the form of an agent liquid, as many agents can be obtained already in liquid form. Such an agent may be in particular an agent that is releasable from the coating in aqueous environments. This has the advantage that such an agent can be released from the coating into an oral cavity of a user that is wearing a dental appliance formed from the functional foil.

Another highly preferred example of the process suggests that the agent is derived from a bio-based material. In other words, the agent may be derived from a renewable biological resource, in particular a material of non-fossil origin produced by a living organism. The term "bio-based material" may be understood here in the sense that a bio-based material may comprise a non-fossil carbon content containing a detectable portion of the carbon isotope ¹⁴C. Preferably, the non-fossil carbon content may constitute at least 20%, preferably at least 35%, most preferably more than 50% of the bio-based material. In other words, about 80% to 65% but preferably much less of the bio-based material can be based on other organic material, in particular polymers, derived from fossil sources such as crude oil.

As will be explained later, such a bio-based material may also be used as the material for the coating and/or such a bio-based material may fully cover a core of a dental appliance fabricated from said functional foil, with the core not being made from a bio-based material.

¹⁴C is a radioactive isotope produced in the earth's atmosphere at a concentration of about 1.25.10⁻¹² = 1250 ppq (ppq = part per quadrillion = 10⁻¹⁵) and incorporated into plants by photosynthesis. If a plant dies, it stops exchanging carbon with its environment, and thereafter the amount of ¹⁴C it contains begins to decrease as the ¹⁴C undergoes radioactive decay. The older a sample containing such biomaterial is, the less ¹⁴C remains to be detected. Hence by detecting the presence of ¹⁴C, material containing non-fossil carbon content can be differentiated from material derived from fossil sources. The presence of the ¹⁴C in a material is thus a direct proof that the material is obtained, at least in part, from non-fossil renewable biological sources. This is because organic polymers derived from fossil oil for example do not show any ¹⁴C any more, since the half-time of radioactive ¹⁴C is less than 6.000 years.

"Detectable portion" may be understood here in that the fraction of the ¹⁴C among all C-atoms of the non-fossil carbon content of the bio-based material may be at least 1 ppq, preferably at least 10 ppq or even at least 100 ppq.

The bio-based material may be further characterized in that it comprises a bio-based content of at least 40%, preferably of at least 50%, and wherein said bio-based content includes the non-fossil carbon content containing ¹⁴C described above as well as nitrogen, oxygen and hydrogen bound to the non-fossil carbon content.

In other words, at least part of the coating may be based on one or more bio-based materials. Preferably, however, the coating may be fabricated exclusively from bio-based materials.

Most preferably, the agent may be derived from an essential oil. Such an oil may be extracted directly from non-fossil plants. Such features ensure that the bio-based agent is not harmful to the body of the user of the dental appliance. Moreover, with such an approach, a myriad of different functionalities such as adding flavor or antimicrobial protection may be achieved by relying on natural products that do not lead to harmful contaminations inside the oral cavity.

According to another preferred example, the agent may comprise at least one of the following substances: an essential oil, an extract of an essential oil, or a derivative from the chemical family of phenols, monoterpenols, aldehydes, ketones, oxide terpenes, ethers, monoterpenes, lactone, phthalides, terpenic aldehydes, sestequiterpen alcohols, sestequiterpen, terpene esters, coumarins, in particular a substance such as cinnamaldehyde, lime, thymol, eugenol, linalool, carvacrol, nutmeg, pimenta berry, rosemary, petitgrain, coffee, anise, spearmint.

Further extracts/derivatives obtained from bio-based materials, in particular through extraction from a natural essential oil, that may be used as an agent to be embedded in the coating, are + α, β-pinene, myristicin, methyl eugenol, menthol, terpinene, p-cymene, linalool, myrcene, pinene, β-ocimene, geranial, sabinene, neral, citronellol, linolenic acid, oleic, stearic, himachalene, bisabolene, trans-cinnamaldehyde, methyl salicytate, eucalyptol, trans-anethole, (+)-limonene, (-)-limonene or L-carvone.

A particular advantage of using lime and cinnamaldehyde as agents is that they are not only highly effective antimicrobial agents but also provide a softening and plasticizing effect to the thermoplastic coating.

To further enhance the safety and compliance of dental appliances formed from the functional foil, it is highly preferable, if the organic polymer contained in the carrier liquid is a bio-based material or at least derived from a bio-based material. In other words, the organic polymer (forming the coating) may be derived from a renewable biological resource, in particular a material of non-fossil origin produced by a living organism.

A particular suitable choice for the organic polymer (forming the coating) is a cellulose-based material, in particular a cellulose-based composite. This is because cellulose is a bio-based material that can render the surface of the dental appliance soft, after swelling in water; in addition, cellulose offers favorable chemcial interactions with the mentioned agents to be loaded or reloaded into the CAP layer comprising cellulose based composites.

Suitable materials for fabricating the core of the foil are cellulose acetate, cellulose acetate butyrate, PLA, chitosan or a polyester, a co-polyester, polycarbonate, polyurethane, polypropylene, polyethylene, polypropylene and polyethylene copolymer, acrylic, cyclic block copolymer, polyetheretherketone, polyamide, polyethylene terephthalate, polybutylene terephthalate, polyetherimide, polyethersulfone, polytrimethylene terephthalate or a combination thereof (e.g., a blend of at least two of the listed hard polymeric materials). In some embodiments, the core of the device can include polymeric materials, such as polycarbonate, a co-polyester, a polyester, and polyurethane. Moreover, the core can also be composed of multiple layers, e.g., two or more polymer layers.

Preferably, however, the core should mainly consist of a thermoplastic polymer (to render the foil thermoformable).

Another highly preferred choice is to use cellulose acetate butyrate (CAB) as the organic polymer (i.e. for the coating). In this case, it is highly preferable for good mechanical properties of the thermoplastic functional foil (and dental appliances formed from the foil), if a butyryl content of the CAB is between 15 to 60% by weight.

By providing such features, it can be achieved that the agent embedded into the coating is releasable from the coating, in particular in aqueous environments, for example in the oral cavity. Moreover, it can be achieved by adequate choice of the agent, for example by using cinnamaldehyde as the agent, that the coating provides antimicrobial protection (through the release of an antimicrobial agent). Hence, the agent may be an antimicrobial agent.

To improve the malleability and adaptiveness of the foil during thermoforming, it is of advantage if a glass transition temperature T_{g,coating} of the coating, and/or in particular a glass transition temperature of the organic polymer T_{g,polymer}, is/are in the range of 80-165°C. This is particularly true when using CAB as the organic polymer.

A suitable corresponding melting range of the coating, and in particular said organic polymer, may be from 120-200°C.

Another important factor for achieving satisfying results when thermoforming the functional foil is the number average molecular weight of the organic polymer used for the coating. For optimum results, the number average molecular weight may be more than 12.000 g/mol, preferably more than 20.000 g/mol, and most preferably more than 30.000 g/mol. Such numbers are particularly suited if CAB is chosen as the organic polymer.

Another highly advantageous example of the process introduced at the beginning proposes that the carrier liquid is a carrier solution, i.e., the carrier liquid may be a solution of a solvent and the organic polymer. In this case, it if preferable if the carrier solution is obtained by dissolving the organic polymer in a solvent. This has the advantage that the coating may be solidified on the core by evaporating the solvent from the coating. This approach is of advantage because the solvent can be used to modify the surface of the core, as will be explained later. As already said, the core may actually be a core foil and hence, the carrier solution may be applied to both sides of the core foil uniformly.

For achieving a uniform coating thickness, the coating can be formed by a physical coating process such as spin coating or blade-based coating, or by spray coating or by roll-to-roll coating. Of course, there is a myriad of different processes are available for performing roll-to-roll coating (e.g. air knife coating, gap coating, immersion dip coating, roller coating, etc.) or spray coating (spray painting, thermal spraying, plasma spraying etc.), as examples.

As already mentioned, the solvent of the carrier solution can be used to modify the surface of the core. In fact, the solvent can soften the core's surface by reducing the interaction of molecular chains of the material of the core. As a result, after treating the core with the carrier solution, the core and the coating (or to be more precise, the organic polymer of the coating) can interlock on a nanometer scale, resulting in improved adhesion of the coating on the core. After application of the coating in liquid form onto the core and interaction of the solvent with the core, the solvent may be simply evaporated thus forming a final solid coating.

Such as softening of the core can be achieved, for example, when using acetone as the solvent and PETG as the core material, but there exist other combinations of core materials and suitable solvents, which achieve the same softening effect, as is readily apparent in the literature. The invention suggests to use this effect for enhancing the adhesion of the coating on the core of a functional foil intended for fabricating a dental appliance.

Another approach for improving the mechanical and/or chemical interaction of the coating and the core is to safeguard that a glass transition temperature T_{g,core} of the core and a glass transition temperature T_{g,coating} of the coating differ by less than 80°C, preferably by less than 60°C. Such a material choice will result in enough mobility of the molecular chains of both core and coating such that efficient thermal interlocking can be achieved through thermal fusion (sometimes referred to as "thermal welding") between core and coating during the final thermoforming of the functional foil.

Moreover, in such a case, it can be of advantage, if the glass transition temperature of the coating T_{g,coating} is higher than the glass transition temperature of the core T_{g,core}. Such a material choice will result in a sufficient mechanical stability during thermoforming of the device, as the organic polymer can provide some stability to the foil. For example, PETG, which is a useful material for the core, can have a glass transition temperature of 80°C, and a melting temperature as high as 210°C. Such a core can be combined with a coating based on cellulose acetate butyrate (CAB), and such a CAB-coating may feature a glass transition temperature T_{g,coating} of 120°C or above, but at the same time a relatively low melting temperature T_{m,coating} of 160°C.

Additionally, either the coating should reach its melting temperature T_{m,coating} before the core reaches its melting temperature T_{m,core} or the core should reach its melting temperature before the coating reaches its own. This may be achieved - in particular when considering thermoforming - by safeguarding that a melting temperature of the core T_{m,core} and a melting temperature of the coating T_{m,coating} differ by at least 20°C, preferable at least 40°C, most preferably at least 60°C. As a result, a compound material may be produced at the interface between core and coating during thermoforming of the functional foil, because when for example the coating reaches its melting temperature, the core may still be in the glass transition phase/temperature range (or vice versa). This compound material will thus comprise chains from the organic polymer of the thermoplastic coating and parts of the material used for the thermoplastic core.

Preferable for easy fabrication, however, will be a design, in which the melting temperature of the core T_{m,core} is at least 20°C, preferable at least 40°C, most preferably at least 60°C, higher than the melting temperature of the coating T_{m,coating}. In such a design, the coating will become molten while the core material is still solid or still in the glass transition stage. This is highly beneficial for obtaining a robust process control, when thermoforming the foil.

The solvent used for the carrier solution may comprise at least one of acetone, methyl acetate, ethyl acetate, methylethyl ketone, isopropyl acetate, butyl acetate, ethyl lactate, cyclohexane, diacetone alcohol, butyl lactate or suitable mixtures of these solvents.

Another variant of the process is defined in that the agent itself may be a liquid. In this case, the agent in liquid form (agent liquid) may be simply mixed with the carrier liquid.

Alternatively, the agent may be added to the carrier liquid by dissolving or emulsifying the agent first in an aqueous or oil-based solution and mixing this solution (agent liquid) with the carrier liquid. In such a case, it is preferable for a homogenous distribution of the agent within the final coating, if the solution containing the agent is mixed with the carrier liquid, for example by a stirrer, to form a homogenous carrier solution, prior to forming the coating.

Concerning the ratio between the agent and the organic polymer in the final coating, this ratio may be typically between 0.01/99.99 and 30/70 by weight, for example 2-3g of agent and 7g of organic polymer. For most applications, it will be preferable if the ratio between the agent and the organic polymer is between 0.1/99.9 and 20/80 by weight.

A particular suitable process can be obtained, if the core of the functional foil is made from Polyethylenterephthalat (PET). In particular, the core may be made at least partly from recycled PET. In other words, the thermoplastic material of the core may comprise recycled PET.

For improving the mechanical properties of the functional foil, in particular for reducing the tendency for crack formation, glykol modified Polyethylenterephthalat (PETG) may be chosen as the material for the core. In these cases, a highly suitable solvent for achieving the softening effect described above is acetone.

The process may be further modified in that a natural softening agent, preferably triacetin or polycaprolactone-triol (PCL-T), is added as a plasticizer to the carrier liquid prior to forming the coating. This may be done, preferably, at a volume ratio of less than 40%, preferably of less than 20%, most preferably of less than 10 %.

Finally, the process explained so far may be continued by forming a dental appliance from the functional foil by thermoforming the foil. Importantly, this thermoforming may be done after application of the coating onto the core, as both the core and the coating may be thermoplastic. As a result, after thermoforming of the functional foil, the organic polymer may form a conformal functional coating, preferably fully covering the core of the foil, which then constitutes the core of the dental appliance. Moreover, an agent previously embedded in the coating (as described above) may then provide antimicrobial protection or regenerative functionality for teeth and gingiva or a flavor or simply a nice color to the dental appliance.

An important aspect with regard to the final thermoforming is the thermal stability of the agent. In order to safeguard the proper functionality of the final device, a decomposition temperature of the agent T_{d,agent} should be at least 10°C above the melting temperature T_{m,coating} of the coating (the decomposition temperature T_{d,agent} may thus lie up to 60°C above the glass transition temperature T_{g,coating} of the coating) .

Following the concept outlined above, the afore-mentioned problem may thus be solved also by a dental appliance according to the invention. As described at the beginning, this dental appliance may be an orthodontic appliance or a protective appliance for protection of teeth and/or gingiva, and it is thermoformed from a functional foil fabricated with a process as described herein or as described by one of the claims directed towards such a process.

In such a dental appliance, the agent described above with respect to the functional foil or an agent embedded into the dental appliance (preferably into a cap layer of the appliance) may be releasable from the dental appliance during intra-oral use. In addition or as an alternative, said agent may be reloadable into a cap layer of the dental appliance using a reload-liquid.

According to a particular approach intended for using the dental appliance purely for protection of teeth and gingiva, the dental appliance may consist entirely of the functional foil, which may have a low thickness of less than 400 µm, preferably of less than 300 µm, most preferably of less than 200 µm.

With respect to the properties of the final device, the invention thus proposes a dental appliance which may be described as featuring a core of thermoplastic material, a thermoplastic coating at least partially covering the core, and an agent embedded in the coating and releasable from the coating in aqueous environment. The core, coating , and agent may be chosen and/or designed as has been described in detail above with respect to the fabrication process or as defined by features of one of the sub-claims directed towards this process.

Most preferably, the agent should be embedded in the coating with a surface density of at least 0.05 mg of the agent per cm² surface area of the coating, because this guarantees a release rate sufficient for achieving the desired functionality of the appliance when worn in the mouth. Such a surface density may be easily reached by using a reload-liquid as defined in one of the claims directed towards a use of such a reload-liquid and/or as described in detail further below.

When achieving such a surface density, a release rate of the agent from the dental appliance of at least 1 mg/m² surface area of the coating per hour over a period of 24 hours can be maintained, when the appliance is worn in the mouth; this is particularly true when using a cellulose-based coating and an agent such as Limonene, cinnamaldehyde, methyl Salicylate or trans-anethole. Such a release rate has been found to be adequate in particular when the agent is chosen to provide antimicrobial protection.

In the use case of dental teeth alignment the embedded agent may also serve as a plasticizer, which reduces the brittleness of the thermoformed dental appliance and enables a long-term stable flexibility during a typical wearing period of two weeks (e.g. for each aligner in a series of aligners).

For providing greater flexibility in the choice of the agent or for replenishing the agent in the coating, after the dental appliance has been used multiple times, a reload-liquid can load the agent into the dental appliance, in particular into the coating/cap layer described previously. The reload-liquid can re-load an agent initially present in the coating but consumed through release from the appliance into the mouth again into the coating. Hence, the reload-liquid can be used both for re-loading a dental appliance (which may be designed and/or fabricated as described previously) with a releasable agent. Such a use may be described in that the reload-liquid contains the agent and the dental appliance is immersed in the reload-liquid to load the agent into the dental appliance.

For achieving sufficient loading, it is preferable if the reload-liquid contains a concentration of the agent of at least 0.2 g/l. In this case, a surface density of the agent embedded in the dental appliance of 0.05 mg/cm² can be achieved. This may result in a release rate from the dental appliance, in particular from said coating / said cap layer, of at least 1 mg/m² per hour, when the dental appliance is worn in the mouth during a typical wearing period of at least 24 hours.

In addition, it is also possible to use such a liquid for loading a releasable agent into a thermoplastic functional foil according to the invention, in particular prior to thermoforming.

The reload-liquid may be in the form of an aqueous solution or an oil-based liquid. It can also contain solvents such as ethanol. Such a solvent can reduce the surface tension and help in wetting the appliance with the reload liquid.

A particular convenient way is to obtain the reload-liquid from a liquid precursor comprising the agent (and preferably a surfactant to be described below), which may then be diluted to obtain the reload liquid.

Preferred examples of the present invention shall now be described in more detail, although the present invention is not limited to these examples. With reference to the accompanying drawings, where features with corresponding technical function are referenced with same numerals even when these features differ in shape or design:
- Fig. 1: is a schematic overview of a fabrication process according to the invention,
- Fig. 2: illustrates the definition of non-fossil carbon content and bio-based content used herein to describe bio-based materials,
- Fig. 3: schematically illustrates a method for loading an agent into a dental appliance (not covered by the claims) using a reload-liquid,
- Fig. 4: shows measurement data of concentrations of a particular agent loaded into different thermoplastic materials, and
- Fig. 5: depicts further experimental data of calorimetric measurements.

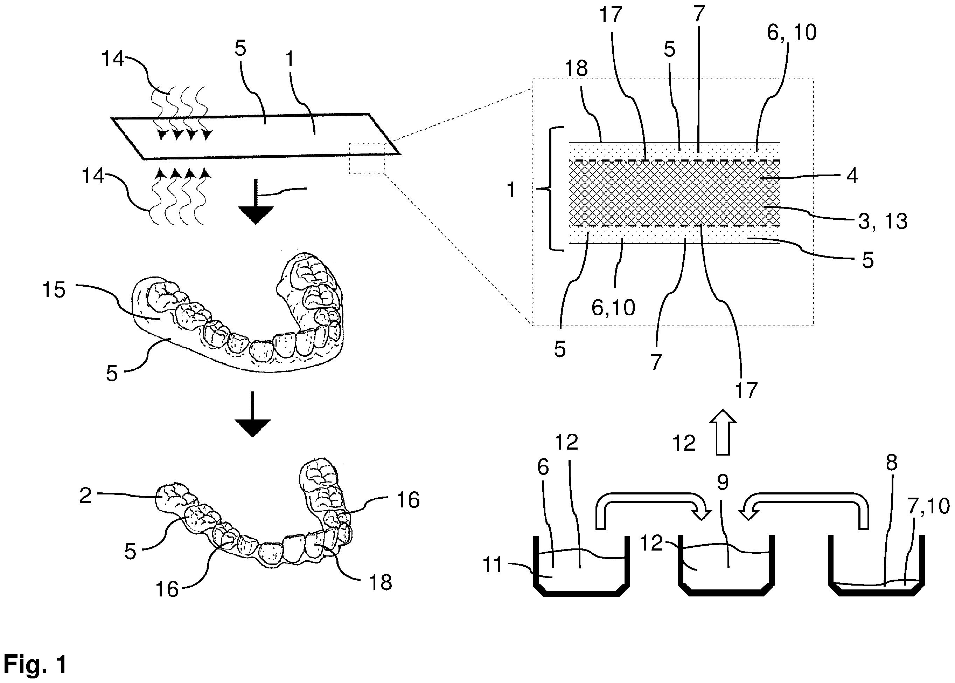

Figure 1 provides an overview of a fabrication process according to the invention: An organic polymer 6, namely cellulose acetate butyrate (CAB), is dissolved in a solvent 12, namely acetone, to form a carrier solution 11. The carrier solution 11 is next mixed with an agent liquid 8 containing cinnamaldehyde, which is a bio-based antimicrobial agent 7, to form a carrier liquid 9, in which the agent 7 and the CAB are homogenously mixed. This carrier liquid 9 is then applied by dip coating onto a core foil 13 of a thermoplastic material 4. After evaporation of the solvent 12, a solid thermoplastic coating 5 is obtained on both sides of the core-foil 13, which thus completely covers the core 3 of the resulting functional foil 1 (c.f. the cross-section visible in Figure 1).

The foil 1, in fact, constitutes a multi-layer structure with a core 3 made from glykol modified Polyethylenterephthalat (PETG) with a thickness of 200 µm and top and bottom functional coatings 5 which each have a thickness (after solidification) of less than 20 µm, thus resulting in a total thickness of the thermoplastic functional foil 1 of less than 250 µm.

As a next step, using a 3D-model of an oral cavity of a patient, a pre-form 15 is formed from the functional foil 1 by thermoforming, applying heat 14 from both sides to the foil 1 and thus distorting the core 3 and the coatings 5 together in one step. In other words, the coating 5 is already firmly linked to the core 3 prior to thermoforming. The underlying reason is that the acetone 12 modifies the surface of the PETG core 3 leading to a softening of the PETG surface such that the CAB molecules 6 can interlock on a nanometer scale at the interface 17 (cf. Figure 1) with the PETG of the core 3. As a result, there is already a high adhesion of the coating 5 on the core 3 prior to thermoforming.

As the glass transition temperatures T_{g,core} of the PETG-core 3 and T_{g,coating} of the coating differ by less than 60°C, during thermoforming the adhesion of the coating 5 on the core foil 13 is further improved, as a thermal interlocking is achieved through thermal fusion of the CAB-coating 5 with the PETG.

Finally, the pre-form 15 is cut such that the resulting dental appliance 2 visible on the bottom left of Figure 1 covers both teeth and adjoining parts of the gingiva. As visible, the dental appliance 2 further features multiple cavities 16 designed for taking up single teeth and which are matching the natural positions of the teeth of the user. Thus, the user experiences no forces on his teeth when wearing the dental appliance 2 on his teeth. As the appliance 2 is very thin, highly conformable and offers a soft and highly deformable functional coating 5, which, due to the CAB used, even softens when getting into contact with saliva and offers antimicrobial protection due to the embedded cinnamaldehyde, the appliance 2 is ideally suited to be worn overnight or while smoking, even for hours. In such non-therapeutic use-cases, the user can benefit from the protection provided by the appliance 2 against cigarette smoke, as the appliance 2 is impermeable to smoke, but also from gingivitis and parodontitis, due to the antimicrobial effect that is produced when the cinnamaldehyde 7 is slowly released from the coating 5 into the oral cavity of the user.

The CAB used for the coating 5 encapsulating the core 3 as illustrated on the right of Figure 1 is in fact a bio-based material 10, as it is produced by blending organic materials derived from fossil sources with organic material, in particular cellulose, derived from non-fossil natural sources such as plants. Therefore, the complete outer surface 18 of the appliance 2 is formed from bio-based materials 10. Any mechanical abrasion from this surface 18 will thus not be harmful for the user wearing the appliance 2 in his mouth.

Likewise, the agent 7 embedded in the coating 5 can be derived from natural sources such as essential oils extracted directly from non-fossil plants. This allows use of agents such as lime, thymol, eugenol, linalool, carvacrol, nutmeg, pimenta berry, rosemary, petitgrain, coffee, anise, to name a few. Hence the appliance 2 can deliver both flavor and antimicrobial protection and still be highly biocompatible and non-harmful to wear.

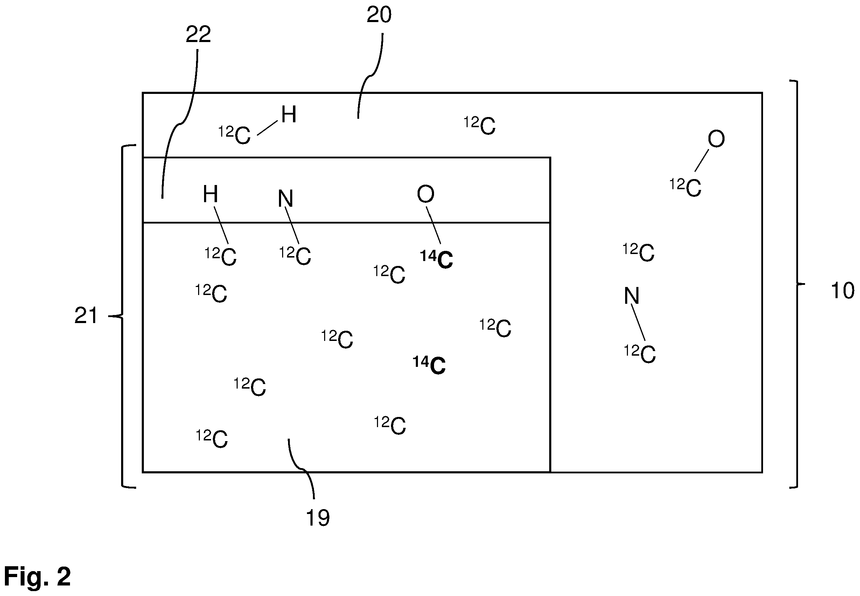

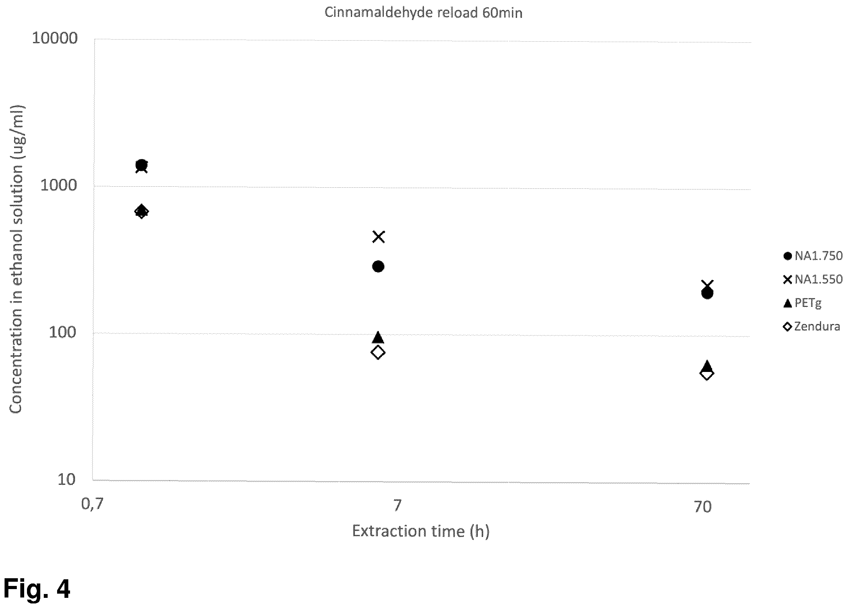

As Figure 2 illustrates schematically, "bio-based material" as understood herein may mean that the organic polymer 6 as well as the agent 7 used for the coating 5 may contain a significant portion of non-fossil carbon content 19, for example at least 40%. This non-fossil carbon content 19 is characterized in that it contains the carbon isotope ¹⁴C in a detectable fraction (e.g. more than 10 ppq or even more than 100 ppq). There may also be a fossil carbon content 20 (cf. Figure 4), which is based on fossil sources and containing the isotope ¹²C but no detectable fraction of ¹⁴C any more, as the ¹⁴C has been diminished by radioactive decay over thousands of years. As illustrated in Figure 4, there can be defined a further quantity namely the so-called bio-based content 21. This fraction of the material 5 comprises the non-fossil carbon content 19 as well as all hydrogen H-, oxygen O-, and nitrogen N-atoms 22 bound to the non-fossil carbon content 19.

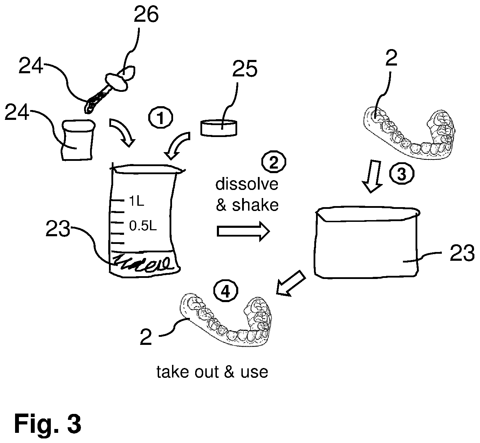

Figure 3 illustrates the formation and use of a reload-liquid: In a first step, a highly concentrated liquid precursor 24 containing the agent 7 and a surfactant are first dispensed in water using a low-cost pipette 26; alternatively, a tab 25 containing the agent 7 and a surfactant may be dissolved in the water. By both ways, a reload-liquid 23 may be formed. In a second step, the reload-liquid 23 is shaken to allow thorough mixing of the liquid precursor 24 with the water and/or full dissolving of the tab 24 in the water. In a third step, the dental appliance 2 is immersed and soaked in the formed reload-liquid 23 for a duration of 15-30 minutes. Finally, in a fourth step, the dental appliance 2 is taken out of the reload-liquid 23 and is now loaded with the agent 7 and ready for use in the mouth, where the agent 7 can be released from the dental appliance 2, for example for producing a pleasant taste and/or an antimicrobial effect.

Figure 4 displays experimental data obtained by first loading four different thermoplastic materials with an agent 7 (cinnamaldehyde) by immersing the respective thermoplastic material in a reload-liquid 23 (comprising 12 g of a surfactant and 0.56 g of the agent 7 dissolved in 100 ml of water) for a time period of 60 min, respectively. Afterwards, the materials were immersed repeatedly in fresh ethanol-solution, which was used as an extraction medium for extracting the agent 7 from the materials, and the concentration of the agent 7 in the respective ethanol-solution was measured each time. In other words, at different points in time, fresh ethanol-solutions were used for extracting the agent from the respective thermoplastic material. The graph displays the measured concentration of the agent 7 in the respective solution for each material for an extraction time of 0.7 h, 7 h, and 70 h, respectively.

As the graph shows (note the logarithmic scale on both axes!), the proposed material system (upper two symbols), comprising a cellulose-based cap layer (materials NA750 and NA550, respectively) applied onto a core of a thermoplastic material, can initially load more of the agent 7 from the same reload-liquid 23 and additionally can store the agent 7 for a much longer period of time (thus offering a lower release rate of the agent 7 over time), as compared to a poly-urethane (PU) based thermofoil (Zendura) or a pure PETG-based thermofoil (both without any coating). In bare numbers, the approach according to the invention allows for an increase in release rate by a factor of 2..6 after a reload time of only 60 minutes; the period of time during which a significant release of the agent is maintained (and thus the desired anti-microbial functionality of the dental appliance) is extended by about six times. The uptake of the cellulose-based cap layers is at least a factor of 2..4 higher, as compared to the pure core materials PETG and PU.

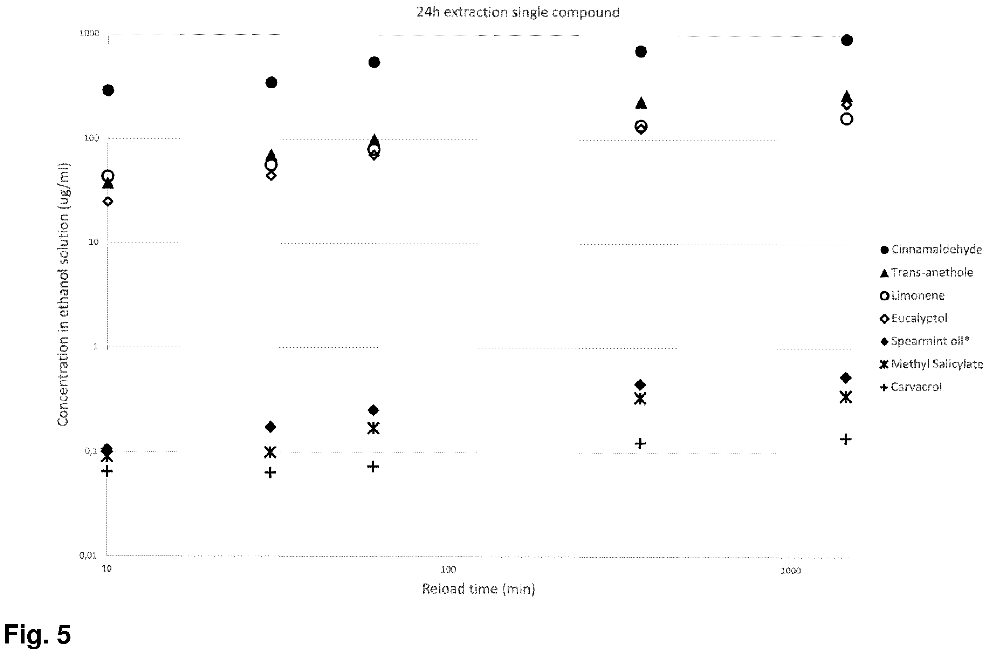

Figure 5 displays experimental data obtained in a second experiment in which dental appliances according to the invention, each time comprising a cellulose-based cap layer, was loaded with 7 different types of agents 7 for time periods ranging from 10 to 1440 min (= 24h). The graph shows the concentration of each agent 7 in 40% ethanol-water-solution, which was used as an extraction medium over a period of 24 hours. Depending on the chemical side group (in particular its polarity) of the particular agent, the capacity to interact with the dental appliance (through storage and release) can vary by a factor of up to 10000, as can be seen by comparing the data for Carvacrol and Cinnamaldehyde. As a result, Cinnamaldehyde appears as an agent that is particularly suited for the applications described herein.

Finally, additional calorimetric experiments were performed to measure the capability of different agents of suppressing the growth of bacteria such as streptococcus mutans and streptococcus mitis. The different agents 7 were applied as a solution containing a single essential oil component at a concentration of 0.1% in BHI. As figure of merits, the lag phase, defined as the postponement in time of the growth of the bacteria and measured in hours, and the growth rate (increase in number of bacteria / time), measured in J/h, of the streptococcus populations were determined as follows:

| agent | growth rate (J/h) of **S. mutans** | lag phase (h) of **S. mutans** growth |
|---|---|---|
| Limonene | 0 | >24 |
| cinnamaldehyde | 0 | >24 |
| trans-anethole | 0.03 | >24 |
| methyl salicylate | 1.2 | 5 |
| Eucalyptol | 1.3 | 5 |

| agent | growth rate (J/h) of **S. mitis** | lag phase (h) of **S. mitis** growth |
|---|---|---|
| methyl salicylate | 0.4 | 23 |
| trans-anethole | 0.13 | 6.4 |
| cinnamaldehyde | 0.15 | 6.2 |
| Limonene | 0.11 | 3 |
| Eucalyptol | 0.14 | 2 |

As can be seen from these data, in particular Limonene and cinnamaldehyde produce lag phases exceeding 24 hours without any measurable growth w.r.t. streptococcus mutans. With respect to streptococcus mitis, methyl salicylate offers the best protection due to a lag phase of 23 hours at a moderate growth rate of 0.4 J/h.

In addition, the difference between non-polar and polar agents should be noted here, since the polarity of the agent 7 will define the reload speed into the cellulose-based (e.g. CAB) coating of the dental appliance 2 as well as the extraction speed in the saliva of the patient. Furthermore, the polarities of the agents 5 impact their respective interaction when combined together in one reload-liquid 23 and thus influence the reload speed of each single agent 7, depending on the ratio to each other and the ratio to the surfactant (if present in the reload-liquid 23).

As cinnamaldehyde is polar, while limonene and methyl salicylate are non-polar, cinnamaldehyde is particularly suited as an anti-microbial agent 7 to be used with a material system as proposed herein with a cellulose-based cap layer, because cinnamaldehyde offers similar anti-microbial protection but superior reload speed.

For achieving best protection against both streptococcus mutans and streptococcus mitis, a combination of Limonene or cinnamaldehyde and methyl salicylate or trans-anethole is proposed to be used as the agent 7 in the reload-liquid 23 described previously and thus as the antimicrobial agent 7 to be delivered by the dental appliance 2 to the user.

In summary, for improving the functionality and biocompatibility of dental appliances 2, a novel process is proposed for making a thermoplastic functional foil 1, from which such dental appliances 2 may be obtained by thermoforming the functional foil 1. A carrier liquid 9 containing an organic and preferably bio-based polymer 6 is enriched with an agent 7 and applied onto a solid thermoplastic core foil 13. After evaporating of a solvent 12 contained in the carrier liquid 9, a uniform and highly homogenous functional thermoplastic coating 5 is obtained on the core foil 13. After thermoforming of the foil 1, the dental appliance 2 thus features an outer protective coating 5 offering enhanced functionality (c.f. Figure 1). Moreover, it is possible to reload the agent 7 into the coating 5 of the appliance 2 using a reload-liquid.

### List of reference numerals

- 1: thermoplastic functional foil
- 2: dental appliance
- 3: core
- 4: thermoplastic material
- 5: thermoplastic coating
- 6: organic polymer
- 7: agent
- 8: agent liquid
- 9: carrier liquid
- 10: bio-based material
- 11: carrier solution
- 12: solvent
- 13: core foil
- 14: heat applied
- 15: pre-form
- 16: cavities (for taking up single teeth)
- 17: interface
- 18: outer surface
- 19: non-fossil carbon content (containing ¹⁴C)
- 20: carbon content based on fossil sources (containing no detectable fraction of ¹⁴C any more)
- 21: bio based content (i.e. non-fossil carbon content plus hydrogen, nitrogen, and oxygen bound to this content)
- 22: hydrogen, nitrogen, and oxygen bound to non-fossil carbon
- 23: reload-liquid
- 24: liquid precursor
- 25: tablet
- 26: pipette

## Claims

1. **Process for making a thermoplastic functional foil (1),** in particular for intra-oral use as a dental appliance (2),
- wherein the functional foil (1) comprises a core (3) of thermoplastic material (4) and a thermoplastic coating (5) which covers the core (3) at least partially, and wherein the process comprises the following steps:
- providing a carrier liquid (9), in particular in the form of a carrier melt, comprising an organic polymer (6);
- adding an agent (7), which can be released from the coating (5) in aqueous environment, to the carrier liquid (9) prior to forming the coating (5); **characterized in that**
- the carrier liquid (9) is applied in the form of a liquid coating (5) onto the solid core (3), which has the form of a core foil (13), and
- the liquid coating (5) is solidified afterwards on the core foil (13) to form a solid thermoplastic coating (5) covering the core (3);

2. Process according to claim 1,
- wherein the agent (7) is derived from a renewable biological resource, in particular a material of non-fossil origin produced by a living organism,
- most preferably from an essential oil, in particular extracted directly from non-fossil plants.

3. Process according to claim 1 or 2,
- wherein the agent (7) comprises at least one of the following substances: an essential oil, an extract of an essential oil, or a derivative such as cinnamaldehyde, lime, thymol, eugenol, linalool, carvacrol, nutmeg, pimenta berry, rosemary, petitgrain, coffee, or anise.

4. Process according to any one of the preceding claims,
- wherein the organic polymer (6) is derived from a renewable biological resource, in particular a material of non-fossil origin produced by a living organism, and/or
- wherein the organic polymer (6) is a cellulose-based material, in particular a cellulose-based composite,
- preferably wherein the organic polymer (6) is cellulose acetate butyrate (CAB),
- most preferably wherein a butyryl content of the CAB is between 15 to 60% by weight.

5. Process according to any one of the preceding claims,
- wherein the coating (5) provides antimicrobial protection, in particular through the release of an antimicrobial agent (7), most preferably cinnamaldehyde.

6. Process according to any one of the preceding claims,
- wherein a melting temperature T_{m,core} of the core (3) and a melting temperature T_{m,coating} of the coating (5) differ by at least 20°C, preferably by at least 40°C,
- most preferably wherein the melting temperature T_{m,core} of the core (3) is higher than the melting temperature T_{m,coating} of the coating (5).

7. Process according to any one of the preceding claims,
- wherein the core (3) is made from Polyethylenterephthalat (PET), in particular at least partly from recycled PET,
- preferably from glykol modified Polyethylenterephthalat (PETG) .

8. Process according to any one of the preceding claims,
- wherein the agent (7) comprises a combination of the following essential oils:
Limonene or cinnamaldehyde
and
methyl salicylate or trans-anethole;

9. Process according to any one of the preceding claims,
- wherein a glass transition temperature T_{g,coating} of the coating (5) is in the range of 80-165°C,
- preferably wherein a decomposition temperature T_{d,agent} of the agent (7) is at least 10°C above a melting temperature T_{m,coating} of the coating (5), and/or
- wherein a corresponding melting range of the coating (5), in particular of said organic polymer (6), is in the range of 120-200°C,
and/or
- wherein the organic polymer (6), in particular the CAB, used for the coating (5) has a number average molecular weight of more than 12.000 g/mol, preferably of more than 20.000 g/mol.

10. Process according to any one of the preceding claims,
- wherein the carrier liquid (9) is a carrier solution (11),
- preferably wherein the carrier solution (11) is obtained by dissolving the organic polymer (6) in a solvent (12),
- most preferably wherein the coating (5) is solidified on the core (3) by evaporating the solvent (12) from the coating (5) and/or
- wherein the core (3) is a core foil (13).

11. Process according to any one of the preceding claims,
- wherein the coating (5) is formed by a physical coating process such as spin coating or blade-based coating, or by spray coating or by roll-to-roll coating.

12. Process according to any one of the preceding claims,
- wherein a glass transition temperature T_{g,core} of the core (3) and a glass transition temperature T_{g,coating} of the coating (5) differ by less than 80°C, preferably by less than 60°C, in particular wherein T_{g,coating} is higher than T_{g,core}.

13. Process according to any one of the claims 10 to 12,
- wherein the solvent (12) modifies the surface of the core (3), in particular by reducing the interaction of molecular chains of the material of the core (3), such that the core (3) and the coating (5) can interlock on a nanometer scale, resulting in improved adhesion of the coating (5) on the core (3), and/or
- wherein the solvent (12) comprises at least one of acetone, methyl acetate, ethyl acetate, methylethyl ketone, isopropyl acetate, butyl acetate, ethyl lactate, cyclohexane, diacetone alcohol, butyl lactate or suitable mixtures of these solvents.

14. Process according to any one of the preceding claims,
- wherein the agent (7) itself is a liquid or
- wherein the agent (7) is added to the carrier liquid (9) by dissolving or emulsifying the agent (7) in an aqueous or oil-based solution and mixing the solution with the carrier liquid (9),
- preferably wherein the solution containing the agent (7) is mixed with the carrier liquid (9), e.g. by a stirrer, to form a homogenous carrier solution (11), prior to forming the coating (5).

15. Process according to any one of the preceding claims,
- wherein a ratio between the agent (7) and the organic polymer (6) is between 0.01/99.99 and 30/70 by weight.

16. Process according to any one of the preceding claims,
- wherein a dental appliance (2) is formed from the functional foil (1) by thermoforming the foil (1) after application of the coating (5) to the core (3),
- in particular such that after thermoforming, the organic polymer (6) forms a conformal functional coating (5), preferably fully covering the core (3),
- in particular wherein the agent (7) embedded in the coating (5) provides antimicrobial or regenerative functionality for teeth and gingiva or a flavor to the dental appliance (2).

17. **Dental appliance** (2), such as an orthodontic appliance (2) or a protective appliance (2) for protection of teeth and/or gingiva, thermoformed from a functional foil (1) **characterized in that**
- the functional foil (1) is fabricated with a process according to any one of the preceding claims,
- preferably wherein the dental appliance (2) consists entirely of the foil (1).

## Patentansprüche

1. **Verfahren zur Herstellung einer thermoplastischen funktionalen Folie (1),** insbesondere zur intraoralen Verwendung als eine Dentalvorrichtung (2),
- wobei die funktionale Folie (1) einen Kern (3) aus thermoplastischem Material (4) und eine thermoplastische Beschichtung (5) umfasst, die den Kern (3) mindestens teilweise bedeckt, und wobei das Verfahren die folgenden Schritte umfasst:
- Bereitstellen einer Trägerflüssigkeit (9), insbesondere in der Form einer Trägerschmelze, die ein organisches Polymer (6) umfasst;
- Hinzufügen eines Mittels (7), das in einer wässrigen Umgebung aus der Beschichtung (5) freigesetzt werden kann, zu der Trägerflüssigkeit (9) vor Formen der Beschichtung (5); **dadurch gekennzeichnet, dass**
- die Trägerflüssigkeit (9) in der Form einer flüssigen Beschichtung (5) auf den festen Kern (3), der die Form einer Kernfolie (13) aufweist, aufgetragen wird und
- die flüssige Beschichtung (5) anschließend auf der Kernfolie (13) ausgehärtet wird, um eine feste thermoplastische Beschichtung (5) zu formen, die den Kern (3) bedeckt.

2. Verfahren nach Anspruch 1,
- wobei das Mittel (7) aus einer erneuerbaren biologischen Ressource, insbesondere einem Material nichtfossiler Herkunft, das durch einen lebenden Organismus hervorgebracht wird,
- am bevorzugtesten aus einem ätherischen Öl, das insbesondere unmittelbar aus nichtfossilen Pflanzen extrahiert ist, abgeleitet ist.

3. Verfahren nach Anspruch 1 oder 2,
- wobei das Mittel (7) mindestens eine der folgenden Substanzen umfasst: ein ätherisches Öl, ein Extrakt eines ätherischen Öls oder ein Derivat wie etwa Zimtaldehyd, Limette, Thymol, Eugenol, Linalool, Carvacrol, Muskatnuss, Pimentbeere, Rosmarin, Petitgrain, Kaffee oder Anis.

4. Verfahren nach einem der vorhergehenden Ansprüche,
- wobei das organische Polymer (6) aus einer erneuerbaren biologischen Ressource, insbesondere einem Material nichtfossiler Herkunft, das durch einen lebenden Organismus hervorgebracht wird, abgeleitet ist, und/oder
- wobei das organische Polymer (6) ein zellulosebasiertes Material, insbesondere ein zellulosebasiertes Verbundprodukt, ist,
- wobei bevorzugt das organische Polymer (6) Zelluloseacetatbutyrat (CAB) ist,
- wobei am bevorzugtesten ein Butyrylgehalt des CAB zwischen 15 und 60 Gewichts-% beträgt.

5. Verfahren nach einem der vorhergehenden Ansprüche,
- wobei die Beschichtung (5) antimikrobiellen Schutz, insbesondere durch die Freigabe eines antimikrobiellen Mittels (7), am bevorzugtesten Zimtaldehyds, bereitstellt.

6. Verfahren nach einem der vorhergehenden Ansprüche,
- wobei sich eine Schmelztemperatur T_{m,core} des Kerns (3) und eine Schmelztemperatur T_{m,coating} der Beschichtung (5) um mindestens 20 °C, bevorzugt um mindestens 40 °C, unterscheiden,
- wobei am bevorzugtesten die Schmelztemperatur T_{m,core} des Kerns (3) höher als die Schmelztemperatur T_{m,coating} der Beschichtung (5) ist.

7. Verfahren nach einem der vorhergehenden Ansprüche,
- wobei der Kern (3) aus Polyethylenterephthalat (PET), insbesondere mindestens teilweise aus wiederverwendetem PET,
- bevorzugt aus Glykol-modifiziertem Polyethylenterephthalat (PETG) hergestellt ist.

8. Verfahren nach einem der vorhergehenden Ansprüche,
- wobei das Mittel (7) eine Kombination der folgenden ätherischen Öle umfasst:
Limonen oder Zimtaldehyd
und
Methylsalicylat oder Transanethol.

9. Verfahren nach einem der vorhergehenden Ansprüche,
- wobei eine Glasübergangstemperatur T_{g,coating} der Beschichtung (5) in dem Bereich von 80-165 °C liegt,
- wobei bevorzugt eine Zersetzungstemperatur T_{d,agent} des Mittels (7) mindestens 10 °C über einer Schmelztemperatur T_{m,coating} der Beschichtung (5) liegt und/oder
- wobei ein entsprechender Schmelzbereich der Beschichtung (5), insbesondere des organischen Polymers (6), in dem Bereich von 120-200 °C liegt
und/oder
- wobei das organische Polymer (6), insbesondere das CAB, das für die Beschichtung (5) verwendet wird, ein zahlenmittleres Molekulargewicht von mehr als 12,000 g/mol, bevorzugt mehr als 20,000 g/mol, aufweist.

10. Verfahren nach einem der vorhergehenden Ansprüche,
- wobei die Trägerflüssigkeit (9) eine Trägerlösung (11) ist,
- wobei bevorzugt die Trägerlösung (11) durch Auflösen des organischen Polymers (6) in einem Lösemittel (12) erlangt wird,
- wobei am bevorzugtesten die Beschichtung (5) auf dem Kern (3) durch Verdampfen des Lösemittels (12) von der Beschichtung (5) ausgehärtet wird und/oder
- wobei der Kern (3) eine Kernfolie (13) ist.

11. Verfahren nach einem der vorhergehenden Ansprüche,
- wobei die Beschichtung (5) durch ein physikalisches Beschichtungsverfahren, wie etwa Schleuderbeschichten oder rakelbasiertes Beschichten, oder durch Sprühbeschichten oder durch Rolle-zu-Rolle-Beschichten geformt wird.

12. Verfahren nach einem der vorhergehenden Ansprüche,
- wobei sich eine Glasübergangstemperatur T_{g,core} des Kerns (3) und eine Glasübergangstemperatur T_{g,coating} der Beschichtung (5) um weniger als 80 °C, bevorzugt um weniger als 60 °C unterscheiden, wobei insbesondere T_{g,coating} höher als T_{g,core} ist.

13. Verfahren nach einem der Ansprüche 10 bis 12,
- wobei das Lösemittel (12) die Oberfläche des Kerns (3) insbesondere durch Reduzieren der Interaktion molekularer Ketten des Materials des Kerns (3) derart modifiziert, dass sich der Kern (3) und die Beschichtung (5) auf einer Nanometerskala gegenseitig verriegeln können, woraus sich eine verbesserte Haftung der Beschichtung (5) auf dem Kern (3) ergibt, und/oder
- wobei das Lösemittel (12) mindestens eines von Aceton, Methylacetat, Ethylacetat, Methylethylketon, Isopropylacetat, Butylacetat, Ethyllactat, Cyclohexan, Diacetonalkohol, Butyllactat oder geeigneten Mischungen dieser Lösemittel umfasst.

14. Verfahren nach einem der vorhergehenden Ansprüche,
- wobei das Mittel (7) selbst eine Flüssigkeit ist oder
- wobei das Mittel (7) durch Auflösen oder Emulgieren des Mittels (7) in einer wässrigen oder ölbasierten Lösung und Mischen der Lösung mit der Trägerflüssigkeit (9) zu der Trägerflüssigkeit (9) hinzugefügt wird,
- wobei bevorzugt die Lösung, die das Mittel (7) enthält, z. B. durch ein Rührwerk mit der Trägerflüssigkeit (9) gemischt wird, um vor dem Formen der Beschichtung (5) eine homogene Trägerlösung (11) zu formen.

15. Verfahren nach einem der vorhergehenden Ansprüche,
- wobei ein Verhältnis zwischen dem Mittel (7) und dem organischen Polymer (6) zwischen 0,01/99,99 und 30/70 nach Gewicht liegt.

16. Verfahren nach einem der vorhergehenden Ansprüche,
- wobei eine Dentalvorrichtung (2) aus der funktionalen Folie (1) durch Thermoformen der Folie (1) nach dem Auftragen der Beschichtung (5) auf den Kern (3) geformt wird,
- insbesondere derart, dass nach dem Thermoformen das organische Polymer (6) eine konforme funktionale Beschichtung (5) formt, die bevorzugt den Kern (3) völlig bedeckt,
- wobei insbesondere das Mittel (7), das in die Beschichtung (5) eingebettet ist, der Dentalvorrichtung (2) eine antimikrobielle oder regenerative Wirkung für Zähne und Zahnfleisch oder einen Geschmack bereitstellt.

17. **Dentalvorrichtung** (2), wie etwa eine zahnregulierende Vorrichtung (2) oder eine Schutzvorrichtung (2) zum Schutz von Zähnen und/oder Zahnfleisch, die aus einer funktionalen Folie (1) thermogeformt ist,
**dadurch gekennzeichnet, dass**
- die funktionale Folie (1) mit einem Verfahren nach einem der vorhergehenden Ansprüche hergestellt ist,
- wobei bevorzugt die Dentalvorrichtung (2) gänzlich aus der Folie (1) besteht.

## Revendications

1. **Procédé de fabrication d'une feuille fonctionnelle (1)** thermoplastique, en particulier pour une utilisation intraorale en tant qu'appareil dentaire (2),
- dans lequel la feuille fonctionnelle (1) comprend un noyau (3) en matériau thermoplastique (4) et un revêtement (5) thermoplastique qui recouvre au moins partiellement le noyau (3), et le procédé comprenant les étapes suivantes :
- la fourniture d'un liquide porteur (9), en particulier sous la forme d'une matière fondue porteuse, comprenant un polymère organique (6) ;
- l'ajout d'un agent (7), qui peut être libéré du revêtement (5) en milieu aqueux, au liquide porteur (9) avant de former le revêtement (5) ; **caractérisé en ce que**
- le liquide porteur (9) est appliqué sous la forme d'un revêtement (5) liquide sur le noyau solide (3), qui a la forme d'une feuille de noyau (13), et
- le revêtement liquide (5) est solidifié ensuite sur la feuille de noyau (13) pour former un revêtement (5) thermoplastique solide recouvrant le noyau (3) ;

2. Procédé selon la revendication 1,
- dans lequel l'agent (7) est issu d'une ressource biologique renouvelable, en particulier d'un matériau d'origine non fossile produit par un organisme vivant,
- le plus préférentiellement d'une huile essentielle, en particulier extraite directement de plantes non fossiles.

3. Procédé selon la revendication 1 ou 2,
- dans lequel l'agent (7) comprend au moins une des substances suivantes : une huile essentielle, un extrait d'huile essentielle, ou un dérivé tel que le cinnamaldéhyde, le citron vert, le thymol, l'eugénol, le linalol, le carvacrol, la noix de muscade, la baie de piment, le romarin, le petit-grain, le café, ou l'anis.

4. Procédé selon l'une quelconque des revendications précédentes,
- dans lequel le polymère organique (6) est issu d'une ressource biologique renouvelable, en particulier d'un matériau d'origine non fossile produit par un organisme vivant, et/ou
- dans lequel le polymère organique (6) est un matériau à base de cellulose, en particulier un composite à base de cellulose,
- de préférence, dans lequel le polymère organique (6) est l'acétate butyrate de cellulose (CAB),
- le plus préférentiellement, dans lequel une teneur en butyryle du CAB est entre 15 et 60 % en poids.

5. Procédé selon l'une quelconque des revendications précédentes,
- dans lequel le revêtement (5) assure une protection antimicrobienne, en particulier par la libération d'un agent antimicrobien (7), le plus préférentiellement le cinnamaldéhyde.

6. Procédé selon l'une quelconque des revendications précédentes,
- dans lequel une température de fusion T_{m,core} du noyau (3) et une température de fusion T_{m,coating} du revêtement (5) diffèrent d'au moins 20 °C, de préférence d'au moins 40 °C,
- le plus préférentiellement, dans lequel la température de fusion T_{m,core} du noyau (3) est supérieure à la température de fusion T_{m,coating} du revêtement (5).

7. Procédé selon l'une quelconque des revendications précédentes,
- dans lequel le noyau (3) est réalisé à partir de polytéréphtalate d'éthylène (PET), en particulier au moins en partie à partir de PET recyclé,
- de préférence à partir de polytéréphtalate d'éthylène modifié par glycol (PETG).

8. Procédé selon l'une quelconque des revendications précédentes,
- dans lequel l'agent (7) comprend une combinaison des huiles essentielles suivantes :
le limonène ou le cinnamaldéhyde
et
le salicylate de méthyle ou le trans-anéthole ;

9. Procédé selon l'une quelconque des revendications précédentes,
- dans lequel une température de transition vitreuse T_{g,coating} du revêtement (5) est dans la plage de 80 à 165 °C,
- de préférence, dans lequel une température de décomposition T_{d,agent} de l'agent (7) est supérieure d'au moins 10 °C à une température de fusion T_{m,coating} du revêtement (5), et/ou
- dans lequel une plage de fusion correspondante du revêtement (5), en particulier dudit polymère organique (6), est dans la plage de 120 à 200 °C, et/ou
- dans lequel le polymère organique (6), en particulier le CAB, utilisé pour le revêtement (5) a un poids moléculaire moyen en nombre de plus de 12 000 g/mol, de préférence de plus de 20 000 g/mol.

10. Procédé selon l'une quelconque des revendications précédentes,
- dans lequel le liquide porteur (9) est une solution porteuse (11),
- de préférence, dans lequel la solution porteuse (11) est obtenue par dissolution du polymère organique (6) dans un solvant (12),
- le plus préférentiellement, dans lequel le revêtement (5) est solidifié sur le noyau (3) par évaporation du solvant (12) du revêtement (5) et/ou
- dans lequel le noyau (3) est une feuille de noyau (13).

11. Procédé selon l'une quelconque des revendications précédentes,
- dans lequel le revêtement (5) est formé par un procédé de revêtement physique tel qu'un revêtement par centrifugation ou un revêtement à la lame, ou par un revêtement par pulvérisation ou par un revêtement rouleau à rouleau.

12. Procédé selon l'une quelconque des revendications précédentes,
- dans lequel une température de transition vitreuse T_{g,core} du noyau (3) et une température de transition vitreuse T_{g,coating} du revêtement (5) diffèrent de moins de 80 °C, de préférence de moins de 60 °C, en particulier dans lequel T_{g,coating} est supérieure à T_{g,core}.

13. Procédé selon l'une quelconque des revendications 10 à 12,
- dans lequel le solvant (12) modifie la surface du noyau (3), en particulier par réduction de l'interaction de chaînes moléculaires du matériau du noyau (3), de telle sorte que le noyau (3) et le revêtement (5) peuvent s'imbriquer sur une échelle nanométrique, ce qui entraîne une adhérence améliorée du revêtement (5) sur le noyau (3), et/ou
- dans lequel le solvant (12) comprend au moins l'un parmi : l'acétone, l'acétate de méthyle, l'acétate d'éthyle, la méthyléthylcétone, l'acétate d'isopropyle, l'acétate de butyle, le lactate d'éthyle, le cyclohexane, l'alcool diacétonique, le lactate de butyle ou des mélanges convenables de ces solvants.

14. Procédé selon l'une quelconque des revendications précédentes,
- dans lequel l'agent (7) est lui-même un liquide ou
- dans lequel l'agent (7) est ajouté au liquide porteur (9) par dissolution ou émulsification de l'agent (7) dans une solution aqueuse ou à base d'huile et par mélange de la solution avec le liquide porteur (9),
- de préférence, dans lequel la solution contenant l'agent (7) est mélangée au liquide porteur (9), par exemple par un agitateur, pour former une solution porteuse (11) homogène, avant de former le revêtement (5).

15. Procédé selon l'une quelconque des revendications précédentes,
- dans lequel un rapport entre l'agent (7) et le polymère organique (6) est entre 0,01/99,99 et 30/70 en poids.

16. Procédé selon l'une quelconque des revendications précédentes,
- dans lequel un appareil dentaire (2) est formé à partir de la feuille fonctionnelle (1) par thermoformage de la feuille (1) après application du revêtement (5) sur le noyau (3),
- en particulier de telle sorte que, après thermoformage, le polymère organique (6) forme un revêtement fonctionnel conforme (5), recouvrant de préférence entièrement le noyau (3),
- en particulier dans lequel l'agent (7) incorporé dans le revêtement (5) fournit une fonctionnalité antimicrobienne ou régénératrice pour les dents et la gencive ou un arôme à l'appareil dentaire (2).

17. **Appareil dentaire** (2), tel qu'un appareil orthodontique (2) ou un appareil de protection (2) pour la protection des dents et/ou de la gencive, thermoformé à partir d'une feuille fonctionnelle (1) **caractérisé en ce que**
- la feuille fonctionnelle (1) est fabriquée avec un procédé selon l'une quelconque des revendications précédentes,
- de préférence, dans lequel l'appareil dentaire (2) est entièrement constitué de la feuille (1).
